# EUROPÄISCHE PATENTANMELDUNG

(11) **EP 1 764 085 A1**
(43) Veröffentlichungstag der Anmeldung: **21.03.2007**
(21) Anmeldenummer: 06120681.9
(22) Anmeldetag: 14.09.2006
(51) Int. Cl.: A61K 8/92, A61P 11/04, A61Q 11/00, A61Q 5/02, A61Q 19/00, A61Q 19/10, A61P 17/00, A61P 29/00, A61P 39/06, A61K 47/44

(54) **Kosmetisches Mittel enthaltend Arganöl**

(30) Priorität: 15.09.2005 DE 102005044186
(71) Anmelder: Aouida, Amin, 67059 Ludwigshafen (DE); Maiwald, Doris, 55767 Abentheuer (DE)
(72) Erfinder: Aouida, M. Amin, 67059, Ludwigshafen (DE)
(74) Vertreter: Reitstötter - Kinzebach

(57) **Zusammenfassung**

Die vorliegende Erfindung betrifft ein kosmetisches Mittel, welches Arganöl enthält.

## Beschreibung

Die vorliegende Erfindung betrifft ein kosmetisches Mittel, welches Arganöl enthält.

Der Arganbaum (lat. Argania spinosa) ist einer der ältesten Bäume der Welt und wächst im südwestlichen Marokko. Der Arganbaum kann mehrmals im Jahr pflaumen- oder olivenähnliche Früchte tragen, die nicht verzehrt werden können. Das Holz, die Blätter und die Früchte des Baumes werden von den Bewohnern Marokkos verwertet und sind sehr wertvoll. Aus den Früchten wird das Arganöl gewonnen, welches in der Kosmetik, Medizin und Gastronomie Anwendung gefunden hat.

Die WO 01/37792 beschreibt dermatologische Zusammensetzungen, die Reisstärke, Kokosprodukte, Karitebutter, Borretschöl, Avocadoöl und Jojobaöl enthalten. Zusätzlich können diese Zusammensetzungen bis zu 1,5 Gew.-% Arganöl enthalten.

Die FR-A-2 756 183 beschreibt eine pharmazeutische oder kosmetische Zusammensetzung, die eine Kombination aus Arganöl und Arganpeptiden enthält.

Überraschenderweise wurde nun gefunden, dass sich hoch Arganöl-haltige Zusammensetzungen besonders vorteilhaft als Naturkosmetika eignen. Dabei kann vorteilhafterweise auf zugesetzte synthetische Konservierungsmittel verzichtet werden.

Gegenstand der Erfindung ist daher ein kosmetisches oder pharmazeutisches Mittel, enthaltend
A) wenigstens 10 Gew.-%, bezogen auf das Gesamtgewicht des Mittels, Arganöl, und
B) wenigstens einen kosmetisch akzeptablen Wirk- und/oder Hilfsstoff,
wobei das Mittel im Wesentlichen frei von synthetischen Konservierungsmitteln ist.

Die erfindungsgemäßen Zusammensetzungen erfüllen vorzugsweise Anforderungen an Naturkosmetik, wie sie beispielsweise vom BDIH (Bundesverband Deutscher Industrie- und Handelsunternehmen für Arzneimittel, Reformwaren, Nahrungsergänzungsmittel und Körperpflegemittel) niedergelegt sind.

Das eingesetzte Arganöl kommt vorzugsweise aus kontrolliert-biologischem Anbau.

Die erfindungsgemäßen Zusammensetzungen enthalten weiterhin vorzugsweise keine Rohstoffe toter Wirbeltiere (z.B. Walrat, Schildkrötenöl, Nerzöl, Murmeltierfett, tierische Fette, tierisches Collagen und Frischzellen).

Im Rahmen der vorliegenden Erfindung bedeutet "im Wesentlichen frei von synthetischen Konservierungsmitteln", dass auf die bewusste Hinzufügung solcher Komponenten verzichtet wird. Der Anteil an diesen Komponenten ist auf die durch Einsatz üblicher Formulierungskomponenten bedingten, nicht zu verhindernden Mengenanteile begrenzt und beträgt insgesamt höchstens 1 Gew.-%, bevorzugt höchstens 0,1 Gew.-%, insbesondere höchstens 0,01 Gew.-% bezogen auf das Gesamtgewicht der Zusammensetzung. Harnstoff ist kein Konservierungsmittel im Sinne der Erfindung.

Die zur Herstellung der Zusammensetzung eingesetzten Komponenten sind insbesondere nicht radioaktiv bestrahlt.

Es wurde gefunden, dass Arganöl antioxidativ wirksame Verbindungen enthält und sich daher in vorteilhafter Weise eignet, schädliche oder unerwünschte oxidative Prozesse, die in der menschlichen Haut ablaufen, zu verringern oder zu vermeiden. Dies trifft im Wesentlichen nur für naturbelassenes Arganöl in hoch Arganöl-haltigen Mitteln zu, die wenigstens 10 Gew.-% Arganöl, bezogen auf das Gesamtgewicht des Mittels, enthalten. Die Verwendung kann dabei sowohl in kosmetischen Mitteln, wie Körperpflegemitteln, Mund- oder Zahnpflegemitteln und Haarbehandlungsmitteln erfolgen, die in der Regel nicht verschreibungspflichtig sind, als auch in Dermatika und pharmazeutischen Mitteln zur Anwendung im Mund- und Rachenraum. Arganöl eignet sich speziell zur Behandlung und Vorbeugung von Stomatitis, Gingivitis, Parodontitis, oralen Muskositis, Laryngitis, Tonsillitis sowie zur Vorbeugung von Karies.

Geeignete kosmetisch akzeptablen Wirk- und/oder Hilfsstoffe B) sind vorzugsweise ausgewählt unter Trägern, Exzipienten, Emulgatoren, Tensiden, natürlichen Konservierungsmitteln, natürlichen Duftstoffen, natürlichen Parfümölen, Verdickern, natürlichen Farbstoffen, Pigmenten, Konsistenzgebern, Entschäumern, Antistatika, Harzen, Lösemitteln, Lösungsvermittlern, Neutralisierungsmitteln, Stabilisatoren, Sterilantien, Treibmitteln, Trocknungsmitteln, Trübungsmitteln, kosmetisch aktiven Wirkstoffen, Haar- und Hautconditionern, Bleichmitteln, Pflegemitteln, Färbemitteln, Tönungsmitteln, Bräunungsmitteln, Feuchthaltemitteln, Rückfettern, Collagen, Eiweißhydrolysaten, Lipiden, Emollienzien, Weichmachern und Mischungen davon.

Eine umfassende Darstellung kosmetischer Hilfsstoffe findet sich in H. P. Fiedler, Lexikon der Hilfsstoffe für Pharmazie, Kosmetik und angrenzende Gebiete, 4. Auflage, Aulendorff: ECV-Editio-Cantor-Verlag, 1996. Eine umfassende Darstellung kosmetischer Grund-, Hilfs- und Wirkstoffe sowie geeignete Formulierungen finden sich weiterhin in K. Schrader, Grundlagen und Rezepturen der Kosmetika, 2. Auflage, Hüthig-Verlag Heidelberg (1989).

Der kosmetisch akzeptable Träger B) ist vorzugsweise ausgewählt unter
i) Wasser,
ii) wassermischbaren organischen Lösungsmitteln, vorzugsweise C₁-C₄-Alkanolen,
iii) natürlichen Ölen, Fetten, Wachsen,
   und Mischungen davon.

Die erfindungsgemäßen Mittel enthalten Arganöl vorzugsweise in einer Menge von wenigstens 15 Gew.-%, bevorzugt von wenigstens 18 Gew.-%, bezogen auf das Gesamtgewicht des Mittels.

Die erfindungsgemäßen Mittel enthalten Arganöl vorzugsweise in einer Menge von höchstens 95 Gew.-%, bevorzugt von höchstens 80 Gew.-%, bezogen auf das Gesamtgewicht des Mittels.

Die erfindungsgemäßen Zusammensetzungen können neben Arganöl weitere Öl-, Fett- und/oder Wachskomponenten B) enthalten. Bevorzugt sind diese weiteren Öl-, Fett- und/oder Wachskomponenten ausgewählt unter pflanzlichen Ölen, Fetten und Wachsen. Insbesondere enthalten die erfindungsgemäßen Zusammensetzungen neben A-gamöl nur pflanzliche Öle, Fette und/oder Wachse. Bevorzugte Öle, Fette und Wachse sind ausgewählt unter Castoröl, Distelöl, Borretschöl, Traubenkernöl, Sojaöl, Erdnussöl, Walnussöl, Haselnussöl, Olivenöl, Baumwollöl, Kürbisöl, Korianderöl, Mohnöl, Kokosnussöl, Kakaoöl, Sonnenblumenöl, Sesamöl, Avocadoöl, Kakaobutter, Mandelöl, Pfirsichkernöl, Sheabutter, Karité-Öl, Calophylumöl, Weizenkeimöl, Macadamianussöl, Nachtkerzenöl, Jojobaöl, Wachsen, wie Bienenwachs, Carnaubawachs, Candilillawachs und Mischungen davon.

Geeignete kosmetisch und pharmazeutisch verträgliche Öl- bzw. Fettkomponenten B) sind in Karl-Heinz Schrader, Grundlagen und Rezepturen der Kosmetika, 2. Auflage, Verlag Hüthig, Heidelberg, S. 319-355 beschrieben, worauf hier Bezug genommen wird.

Vorzugsweise sind die erfindungsgemäßen Mittel im Wesentlichen frei von synthetischen Ölen, Fetten und Wachsen. "Im Wesentlichen frei" bedeutet im Sinne der Erfindung, dass der Anteil an diesen Komponenten insgesamt höchstens 5 Gew.-%, bevorzugt höchstens 1 Gew.-%, insbesondere höchstens 0,5 Gew.-% bezogen auf das Gesamtgewicht der Zusammensetzung enthält. Die erfindungsgemäßen Mittel weisen somit die folgenden Öl-, Fett- und/oder Wachskomponenten nicht auf: Mineralöle und Mineralölprodukte, Paraffin und Paraffinöle, Siliconöle, tierische Öl-, Fett- und/oder Wachskomponenten, etc.

Geeignete hydrophile Träger B) sind ausgewählt unter Wasser, Alkoholen, wie Ethanol, n-Propanol, Isopropanol, n-Butanol, tert.-Butanol und Mischungen davon.

Bevorzugt wird als hydrophiler Träger Wasser eingesetzt. Dabei handelt es sich speziell um hochreines Wasser für naturkosmetische Zwecke. Besonders bevorzugt ist der Einsatz von "X-Wasser" der Firma GSK-Natura GmbH, Hermeskeil. Ein solches Wasser, das etwa 99,6 Gew.-% H₂O und etwa 0,4 Gew.- Na-Silikat/Phosphat enthält, führt zu Zusammensetzungen mit besonders langer Haltbarkeit.

Die erfindungsgemäßen Zusammensetzungen können natürliche Konservierungsmittel B), wie diverse Vitamine und Vitaminderivate, sowie die folgenden naturidentischen Konservierungsmittel enthalten:
- Benzoesäure, ihre Salze und Ethylester
- Salicylsäure und ihre Salze
- Sorbinsäure und ihre Salze
- Benzylalkohol

Die erfindungsgemäßen Zusammensetzungen können weiterhin als Komponente B) wenigstens einen für die Herstellung von Naturkosmetika üblichen Bestandteil enthalten, der durch Derivatisierung, z.B. durch Hydrolyse, Hydrierung, Veresterung, Umesterung oder sonstige Spaltungen und Kondensationen, aus folgenden Naturstoffen gewonnen wird:
- Fette, Öle und Wachse
- Lecithine
- Lanolin
- Mono-, Oligo- und Polysaccharide
- Proteine und Lipoproteine

Vorzugsweise sind die erfindungsgemäßen Zusammensetzungen im Wesentlichen frei von:
- organisch-synthetischen Farbstoffen
- synthetischen Duftstoffen und
- ethoxilierten Rohstoffen.

Geeignete kosmetisch und/oder dermatologisch aktive Wirkstoffe B) sind z. B. färbende Wirkstoffe, Haut- und Haarpigmentierungsmittel, Tönungsmittel, Bräunungsmittel, Bleichmittel, Keratin-härtende Stoffe, antimikrobielle Wirkstoffe, Lichtfilterwirkstoffe, hyperemisierend wirkende Stoffe, keratolytisch und keratoplastisch wirkende Stoffe, Antischuppenwirkstoffe, Antiphlogistika, keratinisierend wirkende Stoffe, hautbefeuchtende oder -feuchthaltende Stoffe, rückfettende Wirkstoffe, antierythimatös oder antiallergisch aktive Wirkstoffe und Mischungen davon.

Die in den erfindungsgemäßen Zusammensetzungen eingesetzten Duftstoffe B) erfüllen vorzugsweise die Zulassungskriterien für natürliche Riechstoffe der ISO-Norm 9235.

Geeignet sind weiterhin Pigmente, wie Titandioxid, Talkum und Zinkoxid.

Geeignet sind weiterhin ätherische Öle, wie Latschenkiefer, Tannenöl, Sandelholz, Teebaum, Lavendel, Rosmarin, Wacholderbeer, Rosskastanienextrakt, Birkenblätterextrakt, Heublumenextrakt, Campher, Menthol, Pfefferminzöl, Nanaminze, Rosmarinextrakt, Eukalyptusöl, Lemongras, Lotusblüte, Zimt, Vanille, Myrte, Wintergrün, Patschouli, Nelkenöl, Palmarosaöl, Ylang Ylang Öl, Rosenöl, Mandarinenöl, Grapefruitöl, Zitronenöl, Jasminöl, Neroli, Blutorangenöl etc. und Gemische davon.

Geeignete Inhaltsstoffe B), die Hautreizungen entgegenwirken, sind z. B. Allantoin, Bisabolol, Dragosantol, Kamillenextrakt, Panthenol, etc.

Die erfindungsgemäßen Mittel können auch pharmazeutisch akzeptable Hilfsstoffe B) enthalten. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB Ph. Eur. BP NF) gelisteten sowie andere Hilfsstoffe, deren Eigenschaften einer physiologischen Anwendung nicht entgegenstehen und wie sie beispielsweise in Fiedler, H. P. Lexikon der Hilfsstoffe für Pharmazie, Kosmetik und angrenzende Gebiete, 4. Aufl., Aulendorf: ECV-Editio-Cantor-Verlag, 1996, dargestellt sind.

Bei den erfindungsgemäßen kosmetischen Mitteln kann es sich um hautkosmetische, dermatologische oder haarkosmetische Mittel handeln. Vorzugsweise liegen die erfindungsgemäßen Mittel in Form eines Gels, Schaums, Sprays, einer Salbe, Creme, Emulsion, Suspension, Lotion, Milch, Fluids oder Paste vor.

Nach einer ersten bevorzugten Ausführungsform handelt es sich bei den erfindungsgemäßen Mitteln um ein Hautreinigungsmittel.

Bevorzugte Hautreinigungsmittel sind Seifen, wie Transparentseifen, Luxusseifen, Deoseifen, Cremeseifen, Babyseifen, Hautschutzseifen, Abrasiveseifen und Syndets, pastöse Seifen, Schmierseifen und Waschpasten, flüssige Wasch-, Dusch- und Badepräparate, wie Waschlotionen, Duschbäder und -gele, Schaumbäder, Ölbäder und Scrub-Präparate.

Nach einer weiteren bevorzugten Ausführungsform handelt es sich bei den erfindungsgemäßen Mitteln um kosmetische Mittel zur Pflege und zum Schutz der Haut, Nagelpflegemittel oder Zubereitungen für die dekorative Kosmetik.

Besonders bevorzugt handelt es sich um Hautpflegemittel, Intimpflegemittel, Fußpflegemittel, Lichtschutzmittel, Repellents, Rasiermittel, Haarentfernungsmittel, Antiaknemittel, Make-ups, Mascara, Lippenstifte, Lidschatten, Kajalstifte, Eyeliner, Rouges und Augenbrauenstifte.

Bei den erfindungsgemäßen Hautpflegemitteln handelt es sich insbesondere um W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Je nach Anwendungsgebiet können die erfindungsgemäßen Mittel in einer zur Hautpflege geeigneten Form, wie z. B. als Creme, Schaum, Gel, Stift, Mousse, Milch, Spray (Pumpspray oder treibmittelhaltiger Spray) oder Lotion appliziert werden.

Die hautkosmetischen Zubereitungen können weitere in der Hautkosmetik übliche Wirkstoffe und Hilfsstoffe, wie zuvor beschrieben, enthalten. Dazu zählen vorzugsweise Emulgatoren, natürliche Parfümöle, kosmetische Wirkstoffe wie Phytantriol, Vitamin A, E und C, Retinol, Bisabolol, Panthenol, Bleichmittel, Färbemittel, Tönungsmittel, Bräunungsmittel, Collagen, Stabilisatoren, pH-Wert-Regulatoren, Farbstoffe, Salze, Verdicker, Gelbildner, Konsistenzgeber, Feuchthaltemittel, Rückfetter und weitere übliche Additive.

Bevorzugt als Öl- und Fettkomponenten der hautkosmetischen und dermatologischen Mittel ist Arganöl, gegebenenfalls im Gemisch mit wenigstens einem der zuvor genannten pflanzlichen Öle.

Die Herstellung der kosmetischen oder dermatologischen Zubereitungen erfolgt nach üblichen, dem Fachmann bekannten Verfahren.

Die erfindungsgemäßen Mittel können in Form von Emulsionen, insbesondere als Wasser-in-Öl-(W/O)- oder Öl-in-Wasser(O/W)-Emulsionen, vorliegen.

Die Herstellung von Emulsionen erfolgt nach bekannten Methoden. Die Emulsionen enthalten neben Arganöl in der Regel übliche Bestandteile, wie Fettalkohole, Fettsäureester und insbesondere Fettsäuretriglyceride, Fettsäuren, Lanolin und Derivate davon, natürliche Öle oder Wachse und Emulgatoren in Anwesenheit von Wasser. Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z. B. für eine Hautcreme etc., enthält im Allgemeinen eine wässrige Phase, die mittels eines geeigneten Emulgatorsystems in einer Öl- oder Fettphase emulgiert ist.

Der Anteil des Emulgatorsystems beträgt in diesem Emulsionstyp bevorzugt etwa 4 bis 35 Gew.-%, bezogen auf das Gesamtgewicht der Emulsion. Vorzugsweise beträgt der Anteil der Fettphase etwa 20 bis 60 Gew.-%. Vorzugsweise beträgt der Anteil der wässrigen Phase etwa 20 und 70 %, jeweils bezogen auf das Gesamtgewicht der Emulsion. Bei den Emulgatoren handelt es sich um solche, die in diesem Emulsionstyp üblicherweise verwendet werden. Sie werden z.B. ausgewählt unter: C₁₂-C₁₈-Sorbitan-Fettsäureestern; Estern von Hydroxystearinsäure und C₁₂-C₃₀-Fettalkoholen; Mono- und Diestern von C₁₂-C₁₈-Fettsäuren und Glycerin oder Polyglycerin; Kondensaten von Ethylenoxid und Propylenglykolen; oxypropylenierten/oxyethylierten C₁₂-C₁₈-Fettalkoholen; polycyclischen Alkoholen, wie Sterolen; aliphatischen Alkoholen mit einem hohen Molekulargewicht, wie Lanolin; Mischungen von oxypropylenierten/polyglycerinierten Alkoholen und Magnesiumisostearat; Succinestern von polyoxyethylenierten oder polyoxypropylenierten Fettalkoholen; und Mischungen von Magnesium-, Calcium-, Lithium-, Zink- oder Aluminiumlanolat und hydriertem Lanolin oder Lanolinalkohol.

Bevorzugte Fettkomponenten, welche in der Fettphase der Emulsionen enthalten sein können, sind neben Arganöl die zuvor genannten Pflanzenöle und Derivate davon. Die Fettphase kann auch in anderen Ölen lösliche Fettsäuren und Fettalkohole enthalten.

Um die Retention von Ölen zu begünstigen, können auch Wachse verwendet werden, wie z. B. Carnaubawachs, Candilillawachs, Bienenwachs, mikrokristallines Wachs, Ozokeritwachs und Ca-, Mg- und Al-Oleate, -Myristate, -Linoleate und -Stearate.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten Arganöl sowie in der Regel Tenside, üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, natürlichen Parfümölen, natürlichen Farbstoffen, organischen Säuren, Saponinen, natürlichen Konservierungsstoffen und sowie Verdickern, Hautkonditioniermitteln und Feuchthaltemitteln.

In den Wasch-, Dusch- und Badepräparaten können alle in Körperreinigungsmitteln üblicherweise eingesetzten anionischen, neutralen, amphoteren oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alky-Iethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten im Molekül aufweisen.

Dazu zählen z. B. Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate, wie Cocodimethylsulfopropylbetain, Laurylbetain, Cocamidopropylbetain oder Natriumcocamphopropionat.

Geeignete kationische Tenside sind z. B. quaternäre Ammoniumverbindungen, beispielsweise Cetyltrimethylammoniumchlorid.

Nach einer weiteren bevorzugten Ausführungsform handelt es sich bei den erfindungsgemäßen Mitteln um ein Haarbehandlungsmittel.

Vorzugsweise liegen die erfindungsgemäßen Haarbehandlungsmittel in Form eines Schaumfestigers, Haarmousses, Haargels, Shampoos, Haarsprays oder Haarschaums vor.

Nach einer weiteren bevorzugten Ausführungsform handelt es sich bei den erfindungsgemäßen Mitteln um ein Mund- oder Zahnpflegemittel. Vorzugsweise liegen diese in Form eines Mundwassers, einer Zahnpasta, eines Zahngels oder eines Gebisspflegemittels vor. Überraschenderweise wurde gefunden, dass Mund- und Zahnpflegemittel, die Arganöl enthalten, über eine gute entzündungshemmende Wirkung verfügen. Der Einsatz von in der Regel unerwünschten Desinfektionsmitteln und Adstringenzien, die die Mundflora mehr schädigen als zu ihrer Gesunderhaltung beizutragen, kann vermieden werden.

Ein übliches erfindungsgemäßes Mundwasser enthält z. B.
10 bis 25. Gew.-% Arganöl,
15 bis 75 Gew.-% Wasser,
15 bis 75 Gew.-% Ethanol,
0 bis 15 Gew.-% Aromaöle,
0 bis 15 Gew.-% weitere Inhaltsstoffe.

Geeignete Aromaöle sind z. B. Menthol, Thymol, Eucalyptol, Eugenol, Anethol etc. Zu den weiteren Inhaltsstoffen zählen z. B. Pflanzenauszüge, wie Kamille, Salbei etc. und Süßstoffe, vorzugsweise frei von Zuckern und synthetischen Zuckerersatzstoffen, wie z. B. aus Stevia rebaudiana (Honigblatt, Süßkraut).

Geeignete Gebisspflegemittel sind Prothesenreinigungsmittel und Haftmittel. Ein übliches Gebissreinigungsmittel enthält neben Arganöl oberflächenaktive Verbindungen, wie z. B. Natriumlaurylsulfat, gegebenenfalls sauerstoffabspaltende Verbindungen, wie Natriumperborat und Kaliummonopersulfat, gegebenenfalls Füllstoffe, wie Natriumcarbonat und/oder Natriumhydrogencarbonat, etc.

Eine bevorzugte Zahnpasta enthält:
15 bis 65 Gew.-% Putzkörper,
15 bis 50 Gew.-% Wasser,
10 bis 25 Gew.-% Arganöl,
10 bis 30 Gew.-% Feuchthaltemittel,
0 bis 2 Gew.-% Bindemittel,
0 bis 25 Gew.-% Ethanol,
0 bis 15 Gew.-% Aromaöle,
0 bis 15 Gew.-% weitere Inhaltsstoffe.

Die in der erfindungsgemäßen Zahnpasta eingesetzten Putzkörper sind vorzugsweise frei von synthetischen Komponenten. Vorzugsweise sind die Putzkörper ausgewählt unter Tonerde, Calciumcarbonat (vorzugsweise in Form von Naturkreide (Schlämmkreide)), Siliciumverbindungen (vorzugsweise Kieselsäure), und Mischungen davon.

Geeignete Feuchthaltemittel sind z.B. Glycerin, Sorbit, Xylit, Polyethylenglykol etc.

Geeignete Bindemittel sind Cellulosederivate, wie Carboxymethylcellulose, Methylcellulose und Hydroxyethylcellulose, Carragheen, Tragant, Alginate, Montmorrilonite, gelbildende Polymere, wie (teil)neutralisierte Acrylsäure, etc.

Geeignete Aromaöle und weitere Inhaltsstoffe sind die zuvor bei den Mundwässern genannten, worauf hier Bezug genommen wird.

Die erfindungsgemäßen Mund- oder Zahnpflegemittel sind im Wesentlichen frei von zugesetzten Fluoriden, synthetischen Tensiden, synthetischen Geschmacks- und Farbstoffen und synthetischen Konservierungsstoffen.

Die Erfindung wird anhand der folgenden nicht einschränkenden Beispiele näher erläutert.

### Beispiele

### Anwendungsbeispiel 1: Körperöl

| INCl-Name | deutsche Bezeichnung | Gew.-% |
|---|---|---|
| Argania spinosa | Arganöl | 20 |
| Citrus nobilis | Mandarinenöl | |
| Cymbopogon martini | Palmarosaöl | |
| Citrus paradisi | Grapefruitöl | |

### Anwendungsbeispiel 2: Gesichtscreme für trockene Haut, Tag- und Nachtcreme

| INCl-Name | deutsche Bezeichnung | Gew.-% |
|---|---|---|
| Aqua | Wasser | |
| Argania spinosa | Arganöl | 20 |
| Hydrogenated Palm Glycerides | gehärtete Palmölglyceride (Tegomuls) | |
| Glyceryl stearate | Glycerinstearat (Lamécreme) | |
| Cetylpalmitate | Cetylpalmitat | |
| Butyrospermum parkii | Sheabutter | |
| Citrus aurantium var. amara | Neroli | |
| Rosa damascena | Rosenöl | |
| Jasminum grandiflorum | Jasminöl | |

### Anwendungsbeispiel 3: Gesichtscreme für fette Haut

| INCl-Name | Rohstoff/Markenname | Gew.-% |
|---|---|---|
| Aqua | Wasser | |
| Argania spinosa | Arganöl | 20 |
| Hydrogenated Palm Glycerides | gehärtete Palmölglyceride (Tegomuls) | |
| Glyceryl stearate | Glycerinstearat (Lamécreme) | |
| Cetylpalmitate | Cetylpalmitat | |
| Butyrospermum parkii | Sheabutter | |
| Laminaria digitata | Fingertang (Algengel) | |
| Citrus aurantium var. amara | Neroli | |
| Rosa damascena | Rosenöl | |
| Mentha viridis | Nanaminze | |

### Anwendungsbeispiel 4: Bodylotion

| INCl-Name | deutsche Bezeichnung | Gew.-% |
|---|---|---|
| Aqua | Wasser | |
| Argania spinosa | Arganöl | 20 |
| Citrus aurantium var. amara | Neroli | |
| Acacia senegal | Gummiarabicum | |
| Cetylpalmitate | Cetylpalmitat | |
| Cymbopogon martinii | Palmarosaöl | |
| Citrus reticulata | Mandarinenöl | |

### Anwendungsbeispiel 5: Hydrophiles Badeöl

| INCl-Name | deutsche Bezeichnung | Gew.-% |
|---|---|---|
| Argania spinosa | Arganöl | 20 |
| Laureth-4 | PEG-4-Laurylether (Mulsifan) | |
| Cananga odorata | Ylang Ylang Öl | |
| Citrus aurantium var. amara | Neroli | |
| Rosa damascena | Rosenöl | |

### Anwendungsbeispiel 6: Shampoo

| INCl-Name | deutsche Bezeichnung | Gew.-% |
|---|---|---|
| Aqua | Wasser | |
| Argania spinosa | Arganöl | 40 |
| Sapindus mukorossi | Waschnuss | |
| Urea | Harnstoff | |
| Citrus aurantium var. amara | Neroli | |
| Acacia senegal | Gummiarabicum | |
| Citrus paradisi | Grapefruitöl | |

### Anwendungsbeispiel 7: Fluid

| INCl-Name | deutsche Bezeichnung | Gew.-% |
|---|---|---|
| Aqua | Wasser | |
| Argania spinosa | Arganöl | 20 |
| Acacia catechu | Gerber-Akazie | |
| Acacia senegal | Gummiarabicum | |
| Citrus aurantium var. amara | Neroli | |
| Rosa damascena | Rosenöl | |
| Jasminum grandiflorum | Jasminöl | |

### Anwendungsbeispiel 8: Reinigungsmilch

| INCl-Name | deutsche Bezeichnung | Gew.-% |
|---|---|---|
| Aqua | Wasser | |
| Argania spinosa | Arganöl | |
| Hydrogenated palm glycerides | gehärtete Palmölglyceride (Tegomuls) | |
| Glyceryl stearate | Glycerinstearat (Lamécreme) | |
| Citrus paradisi | Grapefruitöl | |
| Cymbopogon martini | Palmarosaöl | |

### Anwendungsbeispiel 9: Gesichtswasser

| INCl-Name | deutsche Bezeichnung | Gew.-% |
|---|---|---|
| Aqua | Wasser | |
| Argania spinosa | Arganöl | 20 |
| Urea | Harnstoff | |
| Citrus paradisi | Grapefruitöl | |
| Cymbopogon martini | Palmarosaöl | |

### Anwendungsbeispiel 10: Arganöl-Seife

| INCl-Name | deutsche Bezeichnung | Gew.-% |
|---|---|---|
| Elaeis guineensis* | Palmkernöl | |
| Argania spinosa* | Arganöl | 27 |
| Aqua | Wasser | |
| Potassium carbonate | Kaliumcarbonat | |

| | | |
|---|---|---|
| *) aus kontrolliert biologischem Anbau | | |

### Anwendungsbeispiel 11: Arganöl-Lippenbalsam

| INCl-Name | deutsche Bezeichnung | Gew.-% |
|---|---|---|
| Butyrospermum parkii* | Sheabutter (afrikanischer Butterbaum) | |
| Argania spinosa* | Arganöl | 80 |
| Cera alba | weißes Bienenwachs | |
| Tocopherol | Vitamin E | |
| Carnauba | Carnaubawachs | |

| | | |
|---|---|---|
| *) aus kontrolliert biologischem Anbau | | |

### Anwendungsbeispiel 12: Arganöl-Zahncreme

| INCl-Name | deutsche Bezeichnung | Gew.-% |
|---|---|---|
| Calcium carbonicum | Kreide | |
| Aqua salvia officinalis* | Salbeiwasser | |
| Sorbitol | | |
| Glycerin | | |
| Clay illite | Ton (Illite) | |
| Argania spinosa | Arganöl | 10 |
| Alkohol | Ethanol | |
| Salvia lavendulifolia* | spanischer Salbei | |
| Krameria trianda* | rote Rotanhia | |
| Propolis cera | Propolis-Extrakt | |
| Silica | Kieselsäure | |
| Xanthan gum | Xanthangummi | |
| Coco glucoside | | |
| Titan dioxide | Titandioxid | |
| Stevia rebaudiana | Honigblatt | |
| Sal | | |
| Aromaöl | | |
| Linalol* | | |
| Geraniol* | | |
| Eugenol* | | |

| | | |
|---|---|---|
| *) aus kontrolliert biologischem Anbau | | |

## Patentansprüche

1. Kosmetisches oder pharmazeutisches Mittel, enthaltend
A) wenigstens 10 Gew.-%, bezogen auf das Gesamtgewicht des Mittels, Arganöl, und
B) wenigstens einen kosmetisch akzeptablen Wirk- und/oder Hilfsstoff,
wobei das Mittel im Wesentlichen frei von synthetischen Konservierungsmitteln ist.

2. Mittel nach Anspruch 1, das Arganöl in einer Menge von wenigstens 15 Gew.-%, bevorzugt von wenigstens 18 Gew.-%, bezogen auf das Gesamtgewicht des Mittels, enthält.

3. Mittel nach einem der vorhergehenden Ansprüche, das Arganöl in einer Menge von höchstens 95 Gew.-%, bevorzugt von höchstens 85 Gew.-%, bezogen auf das Gesamtgewicht des Mittels, enthält.

4. Mittel nach einem der vorhergehenden Ansprüche, das im Wesentlichen frei von synthetischen Ölen, Fetten und Wachsen ist.

5. Mittel nach einem der vorhergehenden Ansprüche, wobei die Komponente B) wenigsten einen kosmetisch oder pharmazeutisch akzeptablen Träger umfasst, der ausgewählt ist unter
i) Wasser,
ii) wassermischbaren organischen Lösungsmitteln, vorzugsweise C₁-C₄-Alkanolen,
iii) natürlichen Ölen, Fetten, Wachsen,
und Mischungen davon.

6. Mittel nach einem der vorhergehenden Ansprüche in Form eines Gels, Schaums, Sprays, einer Salbe, Creme, Emulsion, Suspension, Lotion, Milch, Fluids oder Paste.

7. Mittel nach einem der vorhergehenden Ansprüche in Form eines Mund- oder Zahnpflegemittels.

8. Verwendung von Arganöl zur Herstellung eines dermatologischen Mittels zur Behandlung von entzündlichen Hauterkrankungen.

9. Verwendung von Arganöl zur Herstellung eines pharmazeutischen Mittels zur Behandlung und Vorbeugung von Stomatitis, Gingivitis, Parodontitis, oralen Mukositis, Tonsillitis und Laryngitis.

10. Verwendung von Arganöl zur Herstellung eines pharmazeutischen Mittels zur Vorbeugung von Karies.
